# EUROPÄISCHE PATENTSCHRIFT

(11) **EP 0 447 824 B1**
(45) Veröffentlichungstag und Bekanntmachung des Hinweises auf die Patenterteilung: **26.07.1995**
(21) Anmeldenummer: 91102448.7
(22) Anmeldetag: 20.02.1991
(51) Int. Cl.: A61B 17/58

(54) **Tibia-Marknagel mit angepasstem Querschnitt**
Intramedullary nail with adapted cross sections
Clou intramedullaire à sections conformeés

(30) Priorität: 20.03.1990 CH 914/90
(43) Veröffentlichungstag der Anmeldung: 25.09.1991
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- EP-A- 0 094 489
- EP-A- 0 332 857
- DE-U- 8 907 443

## Beschreibung

Die Erfindung bezieht sich auf einen Tibia-Marknagel gemäss Oberbegriff des Anspruchs 1.

Ein solcher Nagel ist beispielsweise aus der EP-A1 0 332 857 bekannt.

Für die Versorgung von Unterschenkelfrakturen wird häufig die Marknagelung angewendet. Die Funktion der Marknagelung entspricht einer inneren Schienung des Röhrenknochens. Eine Schienung kann, je nach Methode, aus einem Rohr oder einer Anzahl von Metallstäben (Bündelnagelung) bestehen. Bei der Anwendung eines Rohrnagels, wie sie beispielsweise in der EP-A1 0 332 857 "Tibia-Marknagel zur Behandlung von Unterschenkelfrakturen" beschrieben ist, muss der Markraum des Röhrenknochens durch Auffräsen vorbereitet werden. Durch diesen Auffräsvorgang erreicht man einen Formschluss zwischen Nagel und Knocheninnenwand. Der Knochen ist bei dieser Methode optimal geschient. Die Vorraussetzung zur Anwendung dieser bekannten Methode ist jedoch, dass axiale Kräfte und Torsionskräfte von der Knochenfraktur aufgenommen werden können.

Das Gleiche gilt für die sogenannte Bündelnagelung, bei welcher der nicht aufgefräste Markraum mit langen Metallstäben aufgefüllt wird. Diese Art der Schienung ist sehr stark vom Geschick des einzelnen Chirurgen abhängig, da es nicht einfach ist, den gesamten Markraumquerschnitt mit diesen Metallstäben zu füllen.

Ist keine knöcherne Abstützung der Knochenfraktur selbst vorhanden (Trümmerbrüche, Knochendefekte) muss die sogenannte Verriegelungs-Marknagelung vorgenommen werden. Bei dieser ebenfalls bekannten Art der Marknagelung, bedarf es keiner Markraumpassung. Trotzdem muss in den meisten Fällen - ausser bei langen Trümmerzonen - eine Auffräsung des Markraums vorgenommen werden, da verriegelbare Marknägel - bedingt durch ihre Belastung - einen gewissen Durchmesser aufweisen und um einiges steifer sind.

Rohrförmige Marknägel, beispielsweise gemäss EP-A1 0 332 857, sind in ihrer Form dem aufgefrästen Markraum angepasst.
Rohrnägel ohne Verriegelungsmöglichkeit besitzen üblicherweise einen kleeblattförmigen Querschnitt und sind teilweise oder durchgehend längs geschlitzt. Das hat den Vorteil, dass sich der Nagel im aufgefrästen Markraum radial verspannen kann, wodurch eine erhöhte Stabilität der Fraktur auf einwirkende Rotationskräfte erzielt wird. Dieses Verspannen des Marknagels bedingt jedoch ein masslich exaktes Auffräsen des Markraums, um ein Verklemmen des Marknagels bei dessen Einführung zu verhindern. Bei der Verriegelungsnagelung wird auf die radiale Vorspannung verzichtet, da die Fraktur durch die quer eingebrachten Schrauben (Verriegelung) gesichert ist. Der Querschnitt dieser Marknägel ist deshalb annähernd kreisförmig, damit sie leicht in den aufgefrästen Markraum eingebracht werden können.

Beim Versuch einen rohrförmigen Marknagel in einen nicht aufgefrästen Markraum zu schlagen, bedarf es eines grossen Kraftaufwandes, da der Tibiaquerschnitt nicht dem Querschnitt des Marknagels entspricht. Dabei kann - neben dem Verklemmen des Marknagels - oft ein Bersten des Tibiaschaftes beobachtet werden.

Um die obengenannten Probleme zu vermeiden, kann zwar ein dünnerer Marknagel gewählt werden, doch ist dies nicht immer möglich, da die mechanische Festigkeit von Rohrnägeln naturgemäss limitiert ist. Diese Festigkeitslimite kann leicht erreicht werden, da diese Art von Marknägel keinerlei rotationshemmende Funktion aufweisen, d.h., dass neben den axial einwirkenden Kräften auch Rotationskräfte vollumfänglich von den Verriegelungsschrauben, bzw. Verriegelungsbolzen aufgenommen werden müssen.

Hier will die Erfindung Abhilfe schaffen.
Der Erfindung liegt die Aufgabe zugrunde, einen Marknagel zu schaffen für die Behandlung von Unterschenkelfrakturen sämtlicher Indikationen mit einer optimal an die Anatomie der Markhöhle, insbesondere in antero-posteriorer Richtung, angepassten Form.
Insbesondere wird die Aufgabe gelöst einen Tibia-Marknagel zu schaffen, welcher im proximalen, spongiösen Teil der Tibia eine hohe Rotationsstabilität aufweist und im distalen, kortikalen Teil der Tibia eine möglichst optimale Anpassung an die Geometrie der Markraumhöhlung gewährleistet.

Die Erfindung löst die gestellte Aufgabe mit einem Tibia-Marknagel, welcher die Merkmale des Anspruchs 1 aufweist.

Der erfindungsgemässe, in der antero-posterioren Ebene geknickte Tibia-Marknagel weist im proximalen und im distalen Endteil unterschiedlich ausgestaltete Querschnitte auf, um den ebenfalls unterschiedlichen Anforderungen in diesen beiden Bereichen zu genügen. Dazu ist der proximale Endteil mit einer annähernd quadratischen Querschnittsfläche ausgebildet, was die Rotationsstabilität in diesem vorwiegend spongiösen Teil der Tibia verbessert. Der distale Endteil ist auf der anterioren Seite gleich ausgebildet wie der proximale Endteil, hingegen ist er auf der posterioren Seite sphärisch ausgebildet um sich der Markraumanatomie in diesem Bereich besser anpassen zu können.

Die Längsachsen der beiden in der antero-posterioren Ebene gegeneinander abgeknickten Endteile schliessen einen Winkel α von 5 bis 13°, vorzugsweise von 7 bis 11°, miteinander ein. Auch die absoluten Dimensionen des Marknagels sind in weiten Bereichen - je nach den anatomischen Erfordernissen - variierbar, doch hat es sich als vorteilhaft erwiesen, wenn die Länge des proximalen Endteils 30 - 35 %, vorzugsweise 33 - 34% der Gesamtlänge des Marknagels beträgt.

Die im distalen Bereich sphärisch ausgebildete, posteriore Seite des Marknagels ermöglicht ein leichtes Eindringen des Implantats, welches dank dieser Formgebung leicht an der posterioren Innenwand der Tibia entlang gleiten kann. Die im distalen Bereich trigonal ausgebildete anteriore Seite des Marknagelquerschnitts entspricht derjenigen des unaufgefrästen Tibiamarkraumquerschnitt in diesem Bereich. Neben der Rotationssicherung hat diese Nagelquerschnittsform den Vorteil, dass ein im Querschnitt grösserer Nagel, als im Vergleich zu einem Nagel mit kreisförmigem Querschnitt, verwendet werden kann. Die Gefahr eines Implantatbruches ist deshalb geringer.

Der volle Nagelquerschnitt hat grundsätzlich zwei Vorteile. Einerseits entsteht nach der Implantation des Marknagels kein Hohlraum im Markkanal, der bei der Behandlung von offenen Frakturen zu Problemen führen kann. Andererseits wird der Nagelquerschnitt im Bereich der Verriegelungsbohrungen weniger geschwächt. Der im Markraum entstehende Hohlraum kann bei der Verwendung von Rohrnägeln bei der Behandlung von offenen Frakturen zu Problemen führen, da der Körper eventuell eingebrachte Fremdkörper nicht abbauen kann, was zu einem Infektionsrisiko führt.

Der im Querschnitt angepasste erfindungsgemässe Marknagel kann, dank seiner anatomischen Form, ohne grossen Kraftaufwand in den Markraum eingeschoben werden. Das hat den Vorteil, dass keine zusätzlichen Verletzungen der Tibia, in Bezug auf Weichteilschädigung, Blutversorgung und Fragmentabsprengung, befürchtet werden müssen.

Ein Ausführungsbeispiel der Erfindung, ist in den Figuren dargestellt und wird im folgenden näher beschrieben.
Fig. 1 stellt einen sagittalen Längsschnitt durch den erfindungsgemässen Tibia-Marknagel dar;
Fig. 2 stellt einen Querschnitt längs der Linie II-II in Fig. 1 dar; und
Fig. 3 stellt einen Querschnitt längs der Linie III-III in Fig. 1 dar.

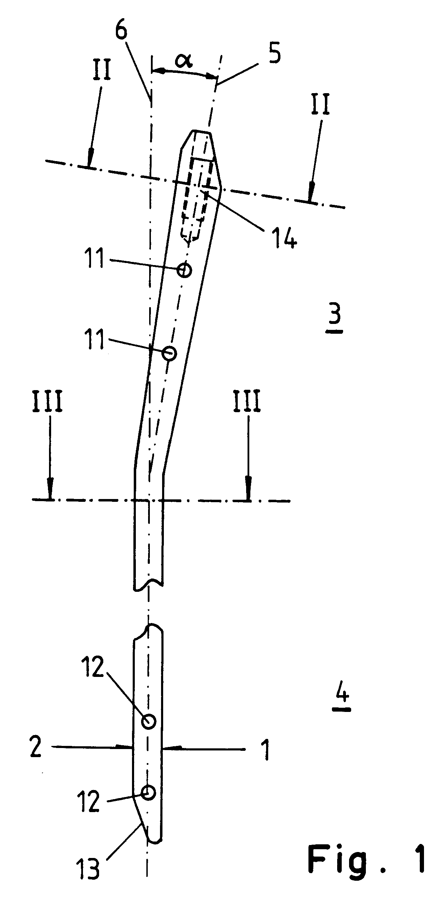

Der in Fig. 1 dargestellte Tibia-Marknagel gemäss der Erfindung ist voll aus einem für solche Implantate üblichen metallischen Werkstoff gefertigt. Er weist eine anteriore Seite 1 und eine posteriore Seite 2 auf und besteht im wesentlichen aus einem proximalen Endteil 3 und einem distalen Endteil 4, welche gegeneinander abgewinkelt sind. Die Längsachsen 5 und 6 der beiden Endteile 3 und 4 schliessen in der sagittalen Ebene - welche der Zeichnungsebene entspricht - einen Winkel α von 8° bis 10°, vorzugsweise von 9° ein.

Der proximale Endteil 3 des Marknagels weist eine Querschnittsfläche 7 auf, welche sowohl in der anterioren Hälfte 1, als auch in der posterioren Hälfte 2 des Marknagels trigonal ausgebildet ist, so dass insgesamt in diesem proximalen Teil ein quadratisches Profil 7 vorliegt.

Der distale Endteil 4 des Marknagels weist eine Querschnittsfläche 8 auf, welche in der anterioren Hälfte 1 ebenfalls trigonal ausgebildet ist - mit einer gegen die anteriore Hälfte 1 hin abgerundeten Spitze - in der posterioren Hälfte 2 jedoch semisphärisch geformt ist.

Im proximalen Endteil 3 sind zwei medio-lateral verlaufende Verriegelungslöcher 11 vorgesehen, welche je eine (nicht dargestellte) Verriegelung-Schraube aufnehmen können. Auch der distale Endteil 4 ist mit zwei medio-lateral verlaufenden Verriegelungslöchern 12 versehen, welche je eine (nicht dargestellte) Verriegelungs-Schraube aufnehmen können.

An seiner proximalen Frontfläche besitzt der proximale Endteil 3 eine Bohrung 14, welche zum Ein- und Ausschlagen des Marknagels mittels dazu geeigneter Instrumente dient.

Schliesslich weist der distale Endteil 4 an seiner Spitze eine nach posterior gerichtete Abstufung 13 auf, welche die problemlose Insertion des Marknagels in den Markraum durch widerstandsarmes Gleiten entlang der dorsalen inneren Kortikalis gestattet.

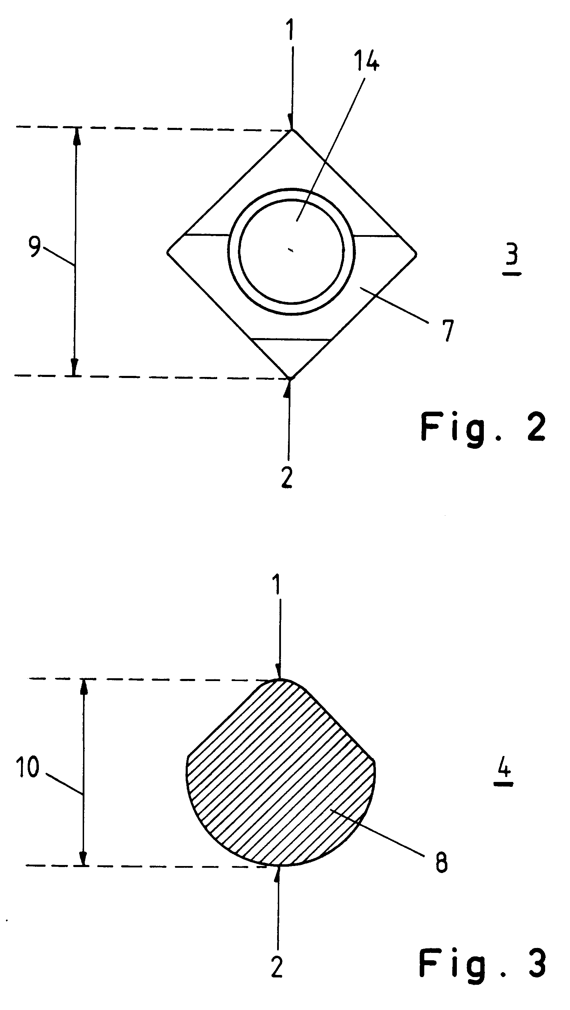

In den Fig. 2 und 3 sind die unterschiedlichen Querschnittsprofile im proximalen 3 und distalen 4 Endteil des Marknagels dargestellt. Das in Fig. 2 gezeigte Profil entspricht der Situation auf Höhe der Schnittlinie II-II in Fig. 1, wobei der in antero-posteriorer Richtung gemessene, maximale Durchmesser 9 des proximalen Endteils 3 von proximal nach distal kontinuierlich abnimmt, vorzugsweise von 12 bis 13 mm auf etwa 8 bis 9 mm.
Das in Fig. 3 gezeigte Profil entspricht der Situation auf Höhe der Schnittlinie III-III in Fig. 1, wobei der in antero-posteriorer Richtung gemessene, maximale Durchmesser 10 des distalen Endteils 4 von proximal nach distal im wesentlichen konstant bleibt.

## Patentansprüche

1. Tibia-Marknagel mit einer anterioren Hälfte (1), einer posterioren Hälfte (2), einem proximalen Endteil (3) und einem distalen Endteil (4), wobei die Längsachsen (5;6) der beiden Endteile (3;4) in der antero-posterioren Ebene einen Winkel von 5 bis 13° miteinander einschliessen, dadurch gekennzeichnet, dass der proximale Endteil (3) eine Querschnittsfläche (7) aufweist, welche sowohl in der anterioren (1), als auch in der posterioren (2) Hälfte trigonal ausgebildet ist und gesamthaft annähernd quadratisch ist, und dass der distale Endteil (4) eine Querschnittsfläche (8) aufweist, welche in der anterioren Hälfte (1) annähernd trigonal und in der posterioren Hälfte (2) annähernd semisphärisch ist.

2. Tibia-Marknagel nach Anspruch 1, dadurch gekennzeichnet, dass die trigonal ausgebildete, anteriore Querschnittsfläche (8) gegen anterior (1) abgerundet ist.

3. Tibia-Marknagel nach Anspruch 1 oder 2, dadurch gekennzeichnet, dass die Länge des proximalen Endteils (3) 30 - 35 %, vorzugsweise 33 - 34% der Gesamtlänge des Marknagels beträgt.

4. Tibia-Marknagel nach einem der Ansprüche 1 bis 3, dadurch gekennzeichnet, dass der in antero-posterioren Richtung gemessene, maximale Durchmesser (9) des proximalen Endteils (3) von proximal nach distal kontinuierlich abnimmt, vorzugsweise von 12 bis 13 mm auf 8 bis 9 mm.

5. Tibia-Marknagel nach einem der Ansprüche 1 bis 4, dadurch gekennzeichnet, dass der in antero-posteriorer Richtung gemessene, maximale Durchmesser (10) des distalen Endteils (4) von proximal nach distal im wesentlichen konstant bleibt.

6. Tibia-Marknagel nach einem der Ansprüche 1 bis 5, dadurch gekennzeichnet, dass der proximale Endteil (3) mindestens ein, vorzugsweise zwei Verriegelungslöcher (11) aufweist.

7. Tibia-Marknagel nach einem der Ansprüche 1 bis 6, dadurch gekennzeichnet, dass der distale Endteil (4) mindestens ein, vorzugsweise zwei Verriegelungslöcher (12) aufweist.

8. Tibia-Marknagel nach Anspruch 6 oder 7, dadurch gekennzeichnet, dass die Verriegelungslöcher (11;12) in medio-lateraler Richtung verlaufen.

9. Tibia-Marknagel nach einem der Ansprüche 1 bis 8, dadurch gekennzeichnet, dass der Marknagel voll ausgebildet ist.

10. Tibia-Marknagel nach einem der Ansprüche 1 bis 9, dadurch gekennzeichnet, dass der distale Endteil (4) an seiner Spitze eine nach posterior gerichtete Abstufung (13) aufweist.

11. Tibia-Marknagel nach einem der Ansprüche 1 bis 10, dadurch gekennzeichnet, dass die Längsachsen (5;6) der beiden Endteile (3;4) in der antero-posterioren Ebene einen Winkel von 7 bis 11°, vorzugsweise von 8 bis 10° miteinander einschliessen.

## Claims

1. Tibia medullary pin with an anterior half (1), a posterior half (2), a proximal end segment (3), and a distal end segment (4), with the longitudinal axes (5;6) of the two end segments (3;4) forming in the antero-posterior plane an angle of from 5° to 13°, characterized in that the proximal end segment (3) has a cross-section area (7) which is trigonal in design in both in the anterior (1) and the posterior (2) half and overall is approximately square, and that the distal end segment (4) has a cross-section area (8) that in the anterior half (1) is approximately trigonal and in the posterior half (2) is approximately semi-spherical.

2. Tibia medullary pin according to claim 1, characterized in that the trigonal anterior cross-section area (8) is rounded off toward the anterior (1).

3. Tibia medullary pin according to claim 1 or 2, characterized in that the length of the proximal end segment (3) is 30 to 35 %, preferably 33 to 34 %, of the entire length of the medullary pin.

4. Tibia medullary pin according to one of claims 1 to 3, characterized in that the maximum diameter (9) of proximal end segment (3), measured in antero-posterior direction, continuously diminishes from proximal to distal, preferably from 12 to 13 mm to 8 to 9 mm.

5. Tibia medullary pin according to one of claims 1 to 4, characterized in that the maximum diameter (10) of the distal end segment (4), measured in antero-posterior direction, remains essentially the same from proximal to distal.

6. Tibia medullary pin according to one of claims 1 to 5, characterized in that the proximal end segment (3) has at least one and preferably two bolt holes (11).

7. Tibia medullary pin according to one of claims 1 to 6, characterized in that the distal end segment (4) has at least one and preferably two bolt holes (12).

8. Tibia medullary pin according to claim 6 or 7, characterized in that the bolt holes (11;12) run in medio-lateral direction.

9. Tibia medullary pin according to one of claims 1 to 8, characterized in that the medullary pin is fully formed.

10. Tibia medullary pin according to one of claims 1 to 9, characterized in that the distal end segment (4) has at its tip a step (13) oriented toward the posterior.

11. Tibia medullary pin according to one of claims 1 to 10, characterized in that the longitudinal axes (5;6) of the two end segments (3;4) form in the antero-posterior plane an angle of from 7° to 11°, preferably from 8° to 10°.

## Revendications

1. Chou médullaire pour tibia comportant une moitié antérieure (1), une moitié postérieure (2), une partie d'extrémité proximale (3) et une partie d'extrémité distale (4), les axes longitudinaux (5; 6) des deux parties d'extrémité (3; 4) formant l'un avec l'autre un angle de 5 à 13° dans le plan antéro-postérieur, caractérisé en ce que la partie d'extrémité proximale (3) présente une section transversale (7) qui est conformée triangulairement aussi bien dans la moitié antérieure (1) que dans la moitié postérieure (2), et est globalement approximativement carrée, et en ce que la partie d'extrémité distale (4) présente une section transversale (8) qui est approximativement en forme de triangle dans la moitié antérieure (1) et approximativement de forme hémisphérique dans la moitié postérieure (2).

2. Chou médullaire pour tibia selon la revendication 1, caractérisé en ce que la section transversale antérieure (8), conformée triangulairement, est arrondie du côté antérieur (1).

3. Chou médullaire pour tibia selon la revendication 1 ou 2, caractérisé en ce que la longueur de la partie d'extrémité proximale (3) vaut 30-35%, de préférence 33-34%, de la longueur totale du chou médullaire.

4. Chou médullaire pour tibia selon l'une quelconque des revendications 1 à 3, caractérisé en ce que le diamètre maximum (9) de la partie d'extrémité proximale (3), mesuré dans la direction antéro-postérieure, diminue de manière continue dans la direction proximaledistale, de préférence depuis 12 à 13 mm jusqu'à 8 à 9 mm.

5. Chou médullaire pour tibia selon l'une quelconque des revendications 1 à 4, caractérisé en ce que le diamètre maximum (10) de la partie d'extrémité distale (4), mesuré dans la direction antéro-postérieure, reste essentiellement constant dans la direction proximale-distale.

6. Chou médullaire pour tibia selon l'une quelconque des revendications 1 à 5, caractérisé en ce que la partie d'extrémité proximale (3) présente au moins un, et de préférence deux trous d'immobilisation (11).

7. Chou médullaire pour tibia selon l'une quelconque des revendications 1 à 6, caractérisé en ce que la partie d'extrémité distale (4) présente au moins un, et de préférence deux trous d'immobilisation (12).

8. Chou médullaire pour tibia pour tibia selon la revendication 6 ou 7, caractérisé en ce que les trous d'immobilisation (11; 12) s'étendent dans la direction médio-latérale.

9. Chou médullaire pour tibia pour tibia selon l'une quelconque des revendications 1 à 8, caractérisé en ce que le chou médullaire est de conformation pleine.

10. Chou médullaire pour tibia selon l'une quelconque des revendications 1 à 9, caractérisé en ce que la partie d'extrémité distale (4) présente à sa pointe un rétrécissement (13) orienté dans la direction postérieure.

11. Chou médullaire pour tibia pour tibia selon l'une quelconque des revendications 1 à 10, caractérisé en ce que les axes longitudinaux (5; 6) des deux parties d'extrémité (3; 4) forment l'un avec l'autre un angle de 7 à 11°, de préférence de 8 à 10° dans le plan antéro-postérieur.
